# EUROPEAN PATENT SPECIFICATION

(11) **EP 4 188 600 B1**
(45) Date of publication and mention of the grant of the patent: **05.06.2024**
(21) Application number: 21746476.7
(22) Date of filing: 20.07.2021
(51) Int. Cl.: B01L 3/00, B01L 7/00

(54) **DEVICE FOR SEPARATION AND/OR PRESERVATION OF A BIOFLUID AT A POINT-OF-CARE, KIT AND METHOD FOR IDENTIFYING A DISEASE**
VORRICHTUNG ZUR TRENNUNG UND/ODER KONSERVIERUNG EINES BIOFLUIDS AN EINEM PFLEGEPUNKT
DISPOSITIF DE SÉPARATION ET/OU DE CONSERVATION D'UN FLUIDE BIOLOGIQUE AU NIVEAU D'UN POINT D'INTERVENTION

(30) Priority: 29.07.2020 EP 20188456
(43) Date of publication of application: 07.06.2023
(73) Proprietor: Max-Planck-Gesellschaft zur Förderung der Wissenschaften e.V., 80539 München (DE)
(72) Inventor: GOMEZ VARELA, David, 37075 Göttingen (DE); STÜHMER, Walter, 37077 Göttingen (DE); KROHN, Markus, 37077 Göttingen/Nikolausberg (DE); BASSU, Marguerita, 8048 Zürich (CH)
(74) Representative: Vossius & Partner Patentanwälte Rechtsanwälte mbB
(86) International application number: PCT/EP2021/070215
(87) International publication number: WO 2022/023114

(56) References cited:
- WO-A1-2009/050435
- WO-A1-2010/109445
- WO-A1-2018/202622
- US-A1- 2010 323 369
- US-B1- 6 372 516

## Description

### BACKGROUND OF THE INVETION

The present invention relates to a device for separation and/or preservation of a biofluid at a point-of-care (for example for separation of plasma or serum from a blood sample and/or for separation of cellular components from saliva), a kit including such device, and a method for identifying a disease.

An earlier device and method allowing to receive a sample at a point-of-care has been described by the inventor in applicant's WO 2018/202622 A1.

However, there is an ongoing need for improved devices and methods.

Other devices are known from US 7,459,125 B1, US 2007/134810 A1, WO 2009/050435 A1, and US 5,766,962 A.

The human race is currently challenged by the fast spread of the coronavirus SARS-CoV-2 that causes COVID-19 (COronaVIrus Disease 2019). Despite the implementation of similar confinement strategies as for SARS (caused by SARS-CoV-1) that emerged in July 2003, COVID-19 has developed into the biggest global pandemic of the century.

Real-time quantitative PCR (RT-qPCR) is the current golden standard for clinical detection of SARS-CoV-2. This testing method is performed on sputum or samples collected in the upper respiratory tract by nasal or throat swabs, which are usually taken by trained personnel. Nevertheless, RT-qPCR tests in infected individuals based on nasal and throat swabs suffer from relatively low reproducibility, which might be explained by the need for a high viral titer in the accumulated mucus of the upper tract, together with variation in the sampling procedure (Zou et.al., SARS-CoV-2 Viral Load in Upper Respiratory Specimens of Infected Patients, N Engl J Med 2020; 382:1177-1179). These drawbacks likely explain the concerning high numbers of false-negative RT-qPCR tests (sensitivity around 60-70%; Ai et.al., id.).

Moreover, it is reported that up to 90% of infected patients have lung lesions (diagnosed by CT scans) before the initial positive RT-qPCR test result (Ai et.al., Correlation of Chest CT and RT-PCR Testing in Coronavirus Disease 2019 (COVID-19) in China: A Report of 1014 Cases, Radiology, published online on February 26, 2020).

The COVID-19 pandemic thus highlights the need for devices and methods allowing for improved point-of-care sampling and testing procedures, as applicable to Sars-CoV-2, but also more generally to other diseases.

### SUMMARY OF THE INVENTION

Without wanting to be bound by theory, it is envisaged by the inventor that the clinical detection of Sars-CoV-2, as well as the clinical detection of other diseases, could be improved by a multi-level analysis of biofluid samples (e.g. blood, saliva). An advantage of blood or saliva samples is that they can be taken and processed in a highly reproducible manner. Moreover, small amounts of these samples (e.g., saliva or capillary blood) can be taken without difficulties at a point-of-care and without particular medical expertise. In particular, such samples could even be taken by the user itself. The device, kit and method according to the invention allow to perform such multi-level analysis on blood and saliva samples taken at a point-of-care.

For example, again without wanting to be bound by theory, it is believed that a possible alternative to the use of nasal or throat swabs for the detection of Sars-CoV-2 would be to use RT-qPCR analysis in a sample that is originally enriched in SARS-CoV-2. Being an RNA virus, SARS-CoV-2 enters cells and uses their cellular replication machinery. Cellular targets of the related SARS-CoV-1 virus are epithelial cells of the lower respiratory tract and lymphocytes in the blood (Wang et.al., Detection and Monitoring of SARS Coronavirus in the Plasma and Peripheral Blood Lymphocytes of Patients with Severe Acute Respiratory Syndrome, Clinical Chemistry, 50:7, 1237-1240). Due to the similarities between these two related viruses (e.g. both viruses cause lymphopenia in most patients), it is assumed by the inventor that lymphocytes (or even cell-free blood plasma) would represent a superior sample for testing purposes than sputum or swabs. It is also known that SARS-CoV-2 can be detected in saliva (Lalli et al., Rapid and extraction-free detection of SARS-CoV-2 from saliva with colorimetric LAMP, https://doi.org/10.1101/2020.05.07.20093542).

It is envisaged by the inventor (again, without wanting to be bound by theory), that an additional strategy could be to analyze lymphocytes with Droplet Digital PCR (dd-PCR), which has shown to be more sensitive to Sars-CoV-2 in pharyngeal swab samples (Suo et.al., ddPCR: a more sensitive and accurate tool for SARS-CoV-2 detection in low viral load specimens, Emerging Microbes & Infections, https://doi.org/10.1101/2020.02. 29.20029439).

Furthermore, it is believed that the analysis of other blood fractions will provide additional insights into the stage and development of the disease.

As will be appreciated, such multi-level analysis is not only helpful in the detection of Sars-CoV-2, but also advantageous for the detection of other diseases (e.g., genetic mutations, for example genetic mutations causing abnormal protein synthesis) and/or other pathogens. Such pathogens include bacteria, a parasite, a fungus, or a different type of virus, such as other coronavirus (e.g., Sars-CoV-1 or MERS) or influenza.

With the device and method according to the present invention, a blood sample (e.g. capillary blood) or a saliva sample may be taken at a point-of-care and may immediately be separated in the device. If necessary, one or more preservatives and/or one or more desiccants can be employed for adequate preservation of one or more separated fractions (i.e., serum or plasma or cellular components present in blood and saliva), thus allowing for shipment of the device to a laboratory where a multi-level analysis can be performed.

More specifically, the device according to the invention is a device for separation and/or preservation of non-cellular components from a biofluid (e.g., for separation of plasma or serum from a blood sample or for separation of the non-cellular components of saliva) at a point-of-care, wherein said device comprises a housing, at least one separation member configured to separate the non-cellular components of the biofluid (e.g., the plasma or serum from the blood sample or the non-cellular components of saliva) while retaining cellular components, and at least one extraction member configured to extract the non-cellular components (e.g., the plasma or serum of the blood sample or the non-cellular components of saliva) from the separation member.

The non-cellular components may also be referred to as a non-cellular liquid fraction of the sample in the context of the present disclosure.

The method according to the invention serves to identify a disease (e.g. a pathogen or a genetic mutation) and comprises the following steps:
(a) a step of providing a device for separation and/or preservation of non-cellular components from a biofluid sample (e.g., for separation of plasma or serum from a blood sample and/or for separation of non-cellular components from saliva) at a point-of-care, wherein said device comprises a housing, at least one separation member configured to separate the non-cellular components from the sample and to retain cellular components, and at least one extraction member configured to extract the non-cellular components from the separation member;
(b) a sample application step of applying the sample to the separation member at the point-of-care;
(c) a separating step of separating, by the separation member, the non-cellular components from the cellular components, wherein the non-cellular components are extracted from the separation member by the extraction member;
(d) an analyzing step of analyzing the separated cellular components and/or the non-cellular components.

The disease may be an infection, in particular a viral infection, a bacterial infection or an infection with a parasite. The disease may also be a genetic mutation, in particular a genetic mutation influencing the synthesis of one or more proteins. As noted above, the virus may be Sars-CoV-2, but could also be another virus, such as another coronavirus or influenza.

As will be noted by the skilled reader, features of the device described hereinafter are applicable to the method and vice versa.

According to the invention, the method preferably comprises a step of adding a preservative for preserving molecular components retained in the separation member. In particular, such preservative may preserve molecular components retained in the separation member, such as RNA, DNA, one or more proteins and/or one or more metabolites retained in the separation member. Such preservative may comprise phenol and/or guanidine. For example, the preservative may comprise TRIzol and/or detergents (e.g. SDS).

The preservative preferably is added after step (c) and before step (d), in particular to the separation member. The preservative preferably is a liquid preservative and may be added in a drop-wise manner.

Preferably, the device is configured to be shifted from a first configuration to a second configuration.

In the first configuration, the device is configured for receiving the sample (e.g., the blood sample or the saliva sample) and/or extracting the non-cellular components (e.g., plasma or serum of the blood sample or the non-cellular components of the saliva sample) from the separation member by the extraction member. This may be achieved by fluidly coupling the separation member and the extraction member to each other in said first configuration.

In the second configuration, the separation member and the extraction member are fluidly uncoupled from each other. This allows the device to receive the preservative in an advantageous manner, in particular a liquid preservative. By fluidly uncoupling the separation member from the extraction member the preservative is prevented from reaching the extraction member and/or a flow assay provided in the device. Without wanting to be bound by theory, it is believed that preservative reaching the non-cellular components (e.g., the plasma or serum of the blood sample or the non-cellular components of the saliva sample) gathered in the extraction member may be detrimental to subsequent testing of the non-cellular components in a multi-level analysis. Shifting the device between the first and second configurations may thus help preventing such contamination.

The separation member may comprise at least one first separation member surface (e.g., a separation member upper surface) onto which the sample is applied and at least one second separation member surface (e.g., a separation member lower surface) from where the non-cellular components (e.g., the plasma or serum of the blood sample or the non-cellular components of the saliva sample) are extracted. In the first configuration, the second separation member surface (e.g., the separation member lower surface) preferably is in contact (e.g., direct contact) with the extraction member, in particular with an extraction member upper surface. In the second configuration, the second separation member surface (e.g., the separation member lower surface) is moved away from and/or not in contact with the extraction member (e.g., with the extraction member upper surface). This may be achieved, for example, by moving the separation member and the extraction member away from each other. Alternatively, a liquid impervious barrier element interrupting the fluid connection could be used. For example, such barrier element could be inserted between the separation member and the extraction member, in particular between the separation member lower surface and the extraction member upper surface.

The device preferably comprises a mechanism for shifting the device between the first configuration and the second configuration. This mechanism may be configured to rotate and/or translate the separation member and the extraction member away from each other when the device is shifted from the first configuration to the second configuration. Alternatively or additionally, the mechanism may be configured to rotate and/or translate the barrier element to arrange it between the separation member and the extraction member.

More specifically, the housing preferably comprises a first housing part and a second housing part. The separation member may be configured to move with the first housing part while the extraction member may be configured to move with the second housing part. For example, the separation member may be fixed to and/or held in the first housing part while the extraction member may be fixed to and/or held in the second housing part. The first housing part may comprise a separation member holder for this purpose, which could be provided by a rim and/or a recess in the first housing part. The second housing part may comprise an extraction member holder, which could be provided by a rim and/or a recess in the second housing part.

The first housing part and the second housing part may be movably connected to each via the mechanism for shifting the device. In particular, the device may be moved between the first configuration and the second configuration by rotating and/or translating the first housing part and the second housing part with respect to each other.

Even more specifically, the mechanism may be configured to rotate the separation member and the extraction member away from each other in a plane (e.g., a horizontal plane). For example, the first housing part may be rotated with respect to a second housing part around an axis of rotation, with the separation member and/or the extraction member being eccentric with respect to said axis. Alternatively or additionally, the mechanism may be configured to translate the separation member and the extraction member away from each other (e.g. in the vertical direction). In either case, the two members will be moved away from each other when performing the rotation and/or translation.

The mechanism may be provided in different manners. For example, a threaded portion may be provided on one of the first housing part and the second housing part, which is engaged with a threaded portion or with one or more protrusions on the other of the first housing part and the second housing part. The mechanism could also be provided by a bayonet mount. For example, one of the first housing part and the second housing part may be provided with one or more protrusions that engage with one or more grooves provided on the other of the first housing part and the second housing part. Each protrusion may then move along a respective groove when the device is shifted from the first configuration to the second configuration.

The one or more grooves may comprise at least one circumferential portion extending along a peripheral sidewall of the respective housing part around the axis of rotation. The circumferential portion may comprise at least one segment extending in a direction having a directional component along the axis of rotation. For example, the least one segment of the circumferential portion may extend obliquely to the axis of rotation, e.g. upwards or downwards. In this manner, a movement of the one or more protrusions along the circumferential segment may cause a translation of the first housing part with respect to the second housing part, for example a translation moving the separation member away from the extraction member in an axial direction. The circumferential portion may include a plurality of such segments. The circumferential portion may thus be formed, at least in part, along a zigzag or undulating line.

The groove may include at least one further portion extending along the axis of rotation. The groove may thus have a generally L or J shape.

Once the device has been shifted from first configuration to the second configuration, the mechanism may hinder the device from being shifted back to the first configuration. This may help to prevent a user from inadvertently contaminating the non-cellular components (e.g., the plasma or serum of the blood sample or the non-cellular components of the saliva sample) in the extraction member after adding preservative to the separation member.

Preferably, the device further comprises at least one flow assay, such as at least one lateral flow assay or at least one vertical flow assay (e.g., an immunoassay). With such flow assay, the method may further comprise at least one flow assay step of applying at least a portion of the sample to the flow assay. Preferably, the portion of the sample is at least a portion of the non-cellular components (e.g., the plasma or serum of the blood sample or the non-cellular components of the saliva sample) extracted by the extraction member. The flow assay step may be at least partially performed (and/or sufficiently performed to yield a result) while the non-cellular components are being separated in the separation step.

The extraction member preferably is fluidly coupled to said flow assay and/or forms a sample receiving section of said flow assay when the device is provided in the first and/or in the second configuration, such that at least part of the non-cellular components gathered in the extraction member is supplied to the flow assay. Providing a fluid connection between the extraction member and the flow assay at least when the device is arranged in the first configuration allows to perform the flow assay concurrently with the separation of non-cellular components. This may reduce the overall time required for receiving a first test result at the point-of-care. The extraction member and the flow assay may be formed as a unitary structure.

Without wanting to be bound by theory, it is believed that such flow assay may further help to avoid false-negative results in the above-mentioned multi-level analysis, e.g. false negative results when performing PCR-based diagnostics. Depending on the circumstances, the use of a flow assay may also provide an immediate response at the point-of-care which may guide a decision regarding further analysis of the sample (e.g., which analytical methods should be used or whether a further analysis is required at all).

The at least one flow assay preferably is configured to identify an infection by the pathogen, e.g. the virus, the bacteria, the parasite, or the fungus. This may be achieved by identifying peptides of the pathogen, e.g. by one or more of nanobodies, and/or antibodies and/or aptamers and/or glycans. Alternatively or additionally, the at least one flow assay may identify a serological response against the pathogen, e.g. the virus, the bacteria, the parasite, or the fungus, in particular IgA and/or IgM and/or IgG antibodies against the pathogen. The pathogen may be a coronavirus or an influenza virus. More particularly, the virus may be Sars-CoV-1, Sars-CoV-2 or MERS.

As known in the art, the at least one flow assay preferably is configured to provide at least one visual response, such as a response including at least one test line and at least one control line. The device may provide, depending on the pathogen, at least one flow assay providing a first test line, a second test line and a control line, or a first test line, a second test line, a third test line and a control line.

As also known in the art, the flow assay may comprise conjugates of an antibody and a detection label, preferably wherein the detection label is a colored material (e.g., Au) or a bioluminescent material.

In order to provide for a more homogeneous speed of molecules (e.g., antibodies) moving through the flow assay, the method may include applying a buffer and/or a detergent to the separation member and/or to the extraction member and/or to the flow assay. This may avoid false negative read-outs (and/or results) of the flow assay.

The extraction member preferably is configured to extract the non-cellular components from the separation member through capillary action. The extraction member may comprise a fibrous material (e.g., a cellulose-based material). For example, the flow assay and/or the extraction member may comprise a cellulose-based material, e.g. nitrocellulose. Alternatively, a micro-structured (i.e., non-fibrous) extraction member may be used.

The separation member preferably is configured to separate the non-cellular components from the sample by gravity and/or capillary action. In particular, the separation member preferably is configured to separate the non-cellular components from the sample without application of pressure and/or suction and/or a centrifugal force. Preferably, no external source of energy is required. This is favorable for performing the separation at the point-of-care. Moreover, this may allow for a better preservation of cellular components in the extraction member and avoid the non-cellular components from being contaminated by the contents of lysed cells.

The separation member may comprise or be composed of a filter (e.g. a serum/plasma separation filter). The filter may have an asymmetric nature wherein cellular components of the sample (e.g. red blood cells, white blood cells, and/or platelets) are captured in larger pores of a first portion of the filter (e.g. an upper portion) while the non-cellular components (e.g., the plasma or serum of the blood sample or the non-cellular components of the saliva sample) flow down into smaller pores in a second portion of said filter (e.g. a lower portion). Alternatively, also a symmetric filter having a defined size cut-off to retain cellular component, e.g. with a size-cutoff of 0.45 micrometer may be employed. Preferred filters are a Vivid GR membrane, a Cobetter filtration membrane and a Primecare^{™} membrane. The Vivid GR membrane and the Primecare^{™} membrane have an asymmetric nature.

The separation of non-cellular components may be performed at the point-of-care. For this purpose, the separation of non-cellular components is preferably performed in less than 30 minutes, in less than 20 min, or even in less than 15 min. For example, sufficient non-cellular components for performing the flow assay and/or for analysis of the non-cellular components in a laboratory preferably is separated within less than 15 minutes, less than 10 minutes, or less than 5 minutes. This allows to minimize the amount of time required at the point-of-care. For example, at least 100 microliter or at least 50 microliter may be separated by the separation member for extraction by the extraction member within less than 15 minutes, less than 10 minutes, or less than 5 minutes. Without wanting to be bound by theory, it is believed that the fast separation of the fractions directly at the point-of-care avoids cross-contamination artifacts, such as the contamination of the cell free fraction by genomic DNA (e.g., when measured as the presence of L1PA2, MSTN, TP53, Actin or GAPDH). In contrast, commercially available formats such as dot-blot-spot (DBS), Volumetric Absorptive Microsampling (VAMS) or Labonovum (blood stored in liquid form) lead to cell rupture and, thereby, contamination of the non-cellular fraction.

The total volume gathered in the extraction member may be 100 microliter or less or even 50 microliter or less. It is envisaged that such volumes would already be sufficient to perform the intended multi-level analysis.

The biofluid sample (e.g., the blood sample or the saliva sample) that may be employed in the device and method according to the present invention may have a volume of 300 microliters or less, 150 microliters or less, 100 microliters or less, or 80 microliters or less. The sample preferably is saliva or whole blood, e.g. capillary blood. The separation of the non-cellular components (e.g., plasma/serum) from the cellular components in such whole blood or saliva sample (as disclosed herein), which allows their separate and effective preservation and/or drying in the device (as also disclosed herein), is considered to be an important aspect of the present invention and particularly advantageous for the subsequent multi-level analysis of the various sample fractions to be performed. Thereby, issues in previous devices and methods, such as cross-contamination of fractions or contamination by lysed cells, etc. can be prevented, while an adequate testing of the different fractions is still possible. The sample preferably is applied as drops, e.g., less than 10 drops or even less than 5 drops of blood (e.g., capillary blood) or saliva.

Alternatively, the device and method according to the present disclosure could also be used for analyzing other types of biological samples, in particular a urine sample or a feces sample (in particular, a buffered feces sample, such as buffered with PBS).

The first housing part preferably comprises a sample introduction portion through which the sample is applied to the separation member. Said sample introduction portion may comprise a through hole. The through hole may be open at a top surface of the sample introduction portion and may extend essentially in the vertical direction. Preferably, the through hole is configured such that the sample can be applied through the through hole onto the separation member when the device is in the first configuration. In particular, the through hole may be disposed above the separation member when the device is in said first configuration. The through hole may allow for direct drop-wise application of blood (e.g., capillary blood), saliva or other biofluids therethrough (e.g., without any further means such as a needle for drawing blood and/or a connection tube connecting such needle to the through hole).

Furthermore, the first housing part may be configured such that the preservative can be applied therethrough to the separation member, e.g. at least when the device is in the second configuration. Preferably, the preservative is applied in a drop-wise manner.

For example, the sample introduction portion may be configured such that the preservative can be applied through the sample introduction portion, e.g. through the through hole, onto the separation member when the device is in the second configuration. In particular, the through hole may be configured such that it is disposed above the separation member when the device is in the first configuration. However, also a further opening could be provided for this purpose, e.g. in the first housing part (in the sample introduction portion or somewhere else).

The sample introduction portion may also comprise one or more air holes. The one or more air holes may have a smaller cross section than the through hole. Such air hole(s) may be used to ensure that air is adequately evacuated from the through hole and/or the separation member when the sample is applied.

The housing (e.g., the first housing part and/or the second housing part) may further include one or more through holes for applying the detergent or buffer to the separation member and/or to the flow assay, e.g. when the device is in the second configuration. As noted above, such buffer and/or detergent may provide for a more homogeneous speed of molecules (e.g., antibodies) moving through the flow assay.

As mentioned above, the second housing part can hold the extraction member and/or the extraction member can be fixed therein. Moreover, the second housing part may also hold the flow assay and/or the flow assay may be fixed therein. In particular, the second housing part may comprise an extraction member holder for fixating the position of the extraction member therein (e.g., when the device is transported and/or shifted between the first and second configuration).

The first housing part and/or the second housing part preferably are configured such that the user and/or patient can identify the result of the flow assay at the point-of-care. In particular, the user preferably is able to see the result of the flow assay without having to open the device, in particular through the first and/or second housing part. For this purpose, the first housing part and/or the second housing part may be made from a transparent material. Alternatively or additionally, the first housing part and/or the second housing part may comprise at least one window through which the flow assay is visible when the first and second housing parts are assembled. Preferably, the window is formed in a bottom of the second housing part, in particular a portion of the bottom that overlaps with the flow assay. The window may be provided as one or more openings in or as a transparent portion of the respective housing part, in particular in the bottom of the second housing part. One or more openings could also be formed as a mesh. The bottom of the second housing part could also be referred to as a base.

Such one or more openings (or, if desired, one or more additional openings), apart from allowing to see the result of the flow assay, may provide further advantages if it is desired to dry the non-cellular components (e.g., the plasma or serum, or the non-cellular components of the saliva) gathered in the extraction member and/or in the flow assay.

More specifically, a desiccant may be used to cover and/or be inserted into such one or more openings. For example, the device may further comprise a desiccant sheet that is used to cover the one or more openings. The sheet may be adhered to the housing part comprising the one or more openings, e.g. to the bottom of the second housing part. For this purpose, the desiccant sheet and/or the bottom of the second housing part may be provided with an adhesive portion, e.g. a portion comprising a pressure sensitive adhesive. Alternatively, the desiccant could be provided as at least one desiccant pellet that is inserted into the one or more openings.

In this manner, the non-cellular components (e.g., the plasma or serum) are gathered in the extraction member and/or in the flow assay would be dried by the desiccant through the one or more openings. As indicated above, such one or more openings preferably overlap with the position of the extraction member and/or with the position of the flow assay. In particular, such one or more openings may be arranged below (e.g., directly below) the extraction member and/or the flow assay. In this manner, the extracted non-cellular components (e.g., the serum or plasma) may be dried without having to dry the entire volume of air in the device. As indicated above, such openings could be provided in conjunction with, but also irrespectively of, the inclusion of a flow assay in the device.

If desired, the desiccant may dry the non-cellular components (e.g., the plasma or serum) in the extraction member through the one or more openings and through the flow assay, for example when the device comprises a vertical flow assay wherein the extraction member is located above the flow assay.

The housing of the device may further comprise a lid. The lid may be connected to the first housing part via a hinge. Said lid may comprise at least one first protrusion configured to extend into the through hole and/or a at least one second protrusion configured to extend into the air hole that may be provided in the sample introduction portion. Preferably, the first and/or the second protrusion extends from a surface of the lid facing towards the first housing part, in particular from a bottom surface of the lid.

The first housing part and/or the second housing part and/or the lid may each be a polymeric component, preferably wherein the first housing part and/or the second housing part and/or the lid are injection molded.

Analyzing the separated cellular components and/or the separated non-cellular components (e.g., the plasma or serum) may comprise a step of analyzing the cellular components and/or the non-cellular components (e.g., the plasma or serum or the non-cellular components of the saliva sample) by genomic technologies in order to identify DNA and/or RNA of the pathogen. This may include, in particular, identifying DNA and/or RNA of the pathogen by polymerase chain reaction (PCR), in particular RT-qPCR and/or dd-PCR. PCR may be used to identify RNA and/or DNA of both the host and/or the pathogen.

For example, the analysis step may include analyzing the cellular components (e.g., the blood cells) retained in the separation member by genomic technologies, such as PCR, in order to identify DNA or RNA of the pathogen (e.g., virus) in these cells. For example, depending on the particular virus, it is envisaged that an analysis of lymphocytes retained in the separation member by PCR (RT-qPCR and/or dd-PCR) will provide for an advantageous detection.

The analysis step may include, in addition or alternatively to PCR, a step of thermal DNA or RNA amplification, such as Loop-mediated isothermal amplification (LAMP) or Reverse Transcription Loop-mediated Isothermal Amplification (RT-LAMP).

The analysis step may include analyzing the cellular components and/or the non-cellular components (e.g., the plasma or serum or the non-cellular components of the saliva sample) by a proteomics-based method, for example by mass spectrometry (e.g., liquid chromatography mass spectrometry). Such analysis may include, in particular, the detection of abnormal protein synthesis or the detection of viral peptides by mass spectrometry. Liquid chromatography mass spectrometry may be particular helpful in this context, but other techniques could be used as well, depending on the particular disease to be identified.

The analysis step may include detecting a cytokine storm by immunofluorescence methods (e.g Luminex technology) in the non-cellular components (e.g., the plasma or serum or the non-cellular components of the saliva sample) and/or in the cellular components. It is believed that this would allow for an early detection of such cytokine storm, which may be particularly dangerous for certain patients (e.g., in the context of Sars-CoV-2).

In order to conserve the non-cellular components (e.g., the plasma or serum or the non-cellular components of the saliva sample) for such testing, it is preferably dried with the above-mentioned desiccant. In order to preserve the cellular components for such testing, they are preferably preserved with the above-mentioned preservative.

Preferably, a multi-level analysis is performed in the analysis step. Such multi-level analysis may include, in particular, analyzing the cellular components retained in the separation member (e.g., by genomic technologies and/or a proteomics-based method such as mass spectrometry and/or by a immunofluorescence method, as discussed above) and analyzing the non-cellular components (e.g., the serum or plasma) gathered in the extraction member and/or in the flow assay (e.g., by genomic technologies and/or a proteomics-based method such as mass spectrometry and/or by a immunofluorescence method, as also discussed above). In particular, the analyzing step may comprise separately analyzing (i) the cellular components, preferably via a first technique (e.g., PCR), and (ii) the non-cellular components (e.g., the plasma or serum), preferably via a second technique (e.g., PCR and/or mass spectrometry). The second technique may be different from the first technique (e.g., a different type of PCR or PCR vs. mass spectrometry).

Since the sample is extracted at the point-of-care, but laboratory equipment will be required for performing the above-mentioned analyses, the method according to the present invention may further comprise a step, after step (c) and before step (d), of transferring the device and/or the separation member and/or the extraction member (preferably the assembled device with the extraction member and the separation member provided therein) to a laboratory. Without wanting to be bound by theory, it is envisaged in view of the above-mentioned drying of the non-cellular components (e.g., the plasma or serum) and/or the above-mentioned preservation of cellular components that this may be done by shipping the device without particular cooling and/or heating (and/or an otherwise controlled environment). The transfer is preferably performed after adding the preservative and/or the desiccant to the device.

According to a further aspect, the invention relates to a kit comprising such device and a preservative for preservation of molecular components retained in the separation member. The preservative may preserve, in particular, RNA, DNA, proteins and/or metabolites contained in the separation member. As noted above, the preservative may comprise phenol and/or guanidine. For example, the preservative may comprise TRIzol and/or detergents (e.g. SDS).

The preservative included in the kit preferably is a liquid. For example, the liquid may be provided in a container from where a determined number of drops can be dispensed without requiring further tools. The preservative could be provided, for example, in a dropper vial, such as a single-dose dropper vial or a bottle with a pipette or dropper nozzle.

The kit may further comprise the above-mentioned detergent and/or buffer for providing a more homogeneous speed of molecules moving through the flow assay. The detergent and/or the buffer are preferably a liquid.

### BRIEF DESCRIPTION OF THE DRAWINGS

The invention will be described in more detail with reference to the figures below. These figures disclose embodiments of the invention for illustrational purposes only. In particular, the disclosure provided by the figures is not meant to limit the scope of protection conferred by the invention.
- Fig. 1: schematically illustrates a method according to an embodiment of the disclosure;
- Fig. 2A: schematically illustrates a separation member and an extraction member in a first configuration;
- Fig. 2B: schematically illustrates the separation member and the extraction member of Fig. 2A being shifted into a second configuration by moving them laterally and/or rotationally with respect to each other;
- Fig. 2C: schematically illustrates the separation member and the extraction member of Fig. 2A being shifted into a second configuration by moving them vertically with respect to each other;
- Fig. 2D: schematically illustrates a second configuration achieved by moving a liquid impervious barrier element between the separation member and the extraction member of Fig. 2A;
- Fig. 3A: schematically illustrates a perspective view of a device according to a first embodiment of the present disclosure;
- Fig. 3B: schematically illustrates a bottom view of a first housing part of the device of Fig. 3A;
- Fig. 3C: schematically illustrates a top view of a second housing part of the device of Fig. 3A;
- Fig. 3D: schematically illustrates a bottom view of the second housing part of the device of Fig. 3A;
- Fig. 4A: schematically illustrates a first possibility of incorporating a desiccant into the device;
- Fig. 4B: schematically illustrates a second possibility of incorporating the desiccant into the device;
- Fig. 5A: schematically illustrates a perspective view of a first housing part of a device according to a second embodiment of the present disclosure;
- Fig. 5B: schematically illustrates a top view of the first housing part of Fig. 5A;
- Fig. 5C: schematically illustrates a cross section along the line B-B indicated in Fig. 5B;
- Fig. 5D: schematically illustrates a perspective view of a second housing part of the device according to the second embodiment;
- Fig. 5E: schematically illustrates a side view of the second housing part of Fig. 5D;
- Fig. 5F: schematically illustrates a perspective view of the second housing part of Figs. 5D and 5E from below;
- Fig. 6: shows graphs illustrating the performance of the device disclosed herein for gathering plasma, white blood cells, and white blood cell subtypes;
- Fig. 7: shows a graph illustrating the levels of L1PA2 DNA in the device disclosed herein and other methods for sampling capillary blood;
- Fig. 8: shows a graph illustrating the detection of anti-SARS-CoV-2 IgG antibodies in the serum of a COVID-19 seroconverted patient.

### DETAILED DESCRIPTION

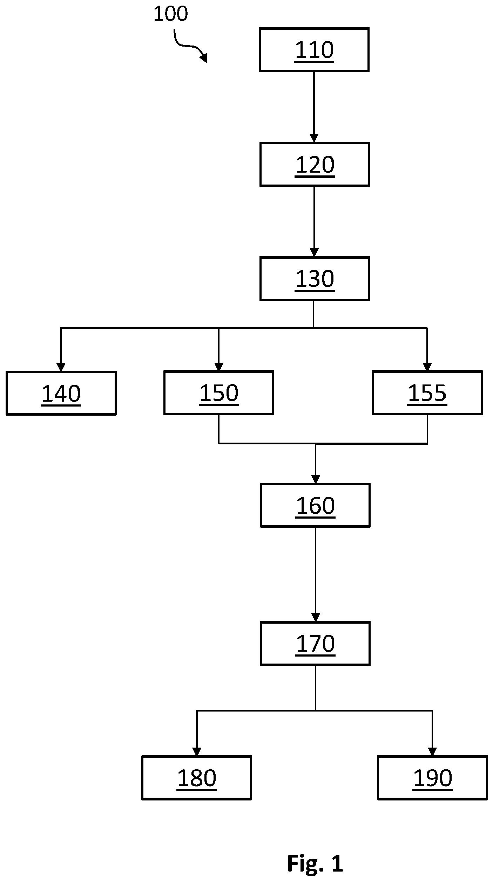

Fig. 1 schematically illustrates a method 100 for identifying a disease - for example an infection with a pathogen (e.g., a viral infection, such as Sars-CoV-2, a bacterial infection or an infection with a parasite) or a genetic mutation (e.g., a genetic mutation influencing the production of one or more proteins) - according to the present invention. The method 100 preferably employs minute amounts of a biofluid as a biological sample (e.g., 80 microliter or less), in particular minute amounts of blood or saliva, for a rapid separation of non-cellular components from a biological sample (e.g., plasma or serum from a blood sample or non-cellular components from a saliva sample) and, preferably, for providing a first analysis result via a flow assay (e.g., in 15 minutes or less). At the same time, the method 100 allows for an accurate characterization of molecular alterations occurring at different molecular levels during the course of the disease in a laboratory.

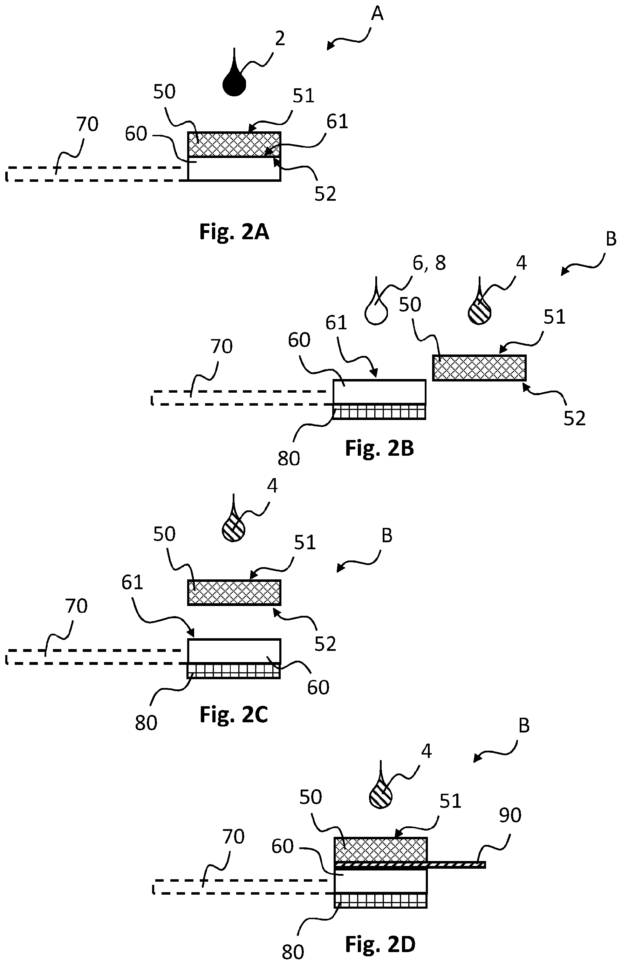

The method 100 includes a first step 110 of providing a device for separation of non-cellular components (e.g., plasma or serum from the blood sample) at a point-of-care. The device comprises a housing, at least one separation member configured to separate the non-cellular components (e.g., the plasma or serum) from the sample and to retain cellular components, and at least one extraction member configured to extract the non-cellular components (e.g., the plasma or serum) from the separation member. Suitable devices and some of their possible functional principles are described in more detail below with reference to Figs. 2 to 5. The sample (e.g., the blood or saliva sample) may be applied to the device as drops, for example as less than 10 drops or less than 5 drops. The blood may be capillary blood.

The method 100 further includes at least one sample application step 120 of applying the sample to the separation member at the point-of-care. After the sample is applied, a separating step 130 of separating, by the separation member, the non-cellular components (e.g., the plasma or serum) from the cellular components, is performed. This is achieved by extracting non-cellular components (e.g., plasma or serum) separated by the separation member by the extraction member provided in the device. The separating step 130 is preferably performed at the point-of-care.

As further indicated in Fig. 1, a flow assay step 140 may be performed at this stage. For this purpose, a flow assay is provided in the device and at least a portion of the sample is applied thereto, preferably at least a portion of the non-cellular components (e.g., the plasma or serum) separated by the separation member. The non-cellular components may flow into the flow assay from the extraction member. As such, the flow assay step 140 may be performed while the plasma or serum is still being separated and extracted in the separating step 130. After the flow assay step 140 has been completed, the result of the flow assay may immediately be read out by the user and/or the patient.

The method may also comprise adding a buffer and/or a detergent to the flow assay in the step 140 in order to provide for a more homogeneous speed of the antibodies moving through the assay. This may be done, for example, after the separating step 130 has been completed. The buffer and/or detergent may be added as a liquid, e.g. drop-wise.

Subsequent to the separation step 130, the method 100 comprises a step 150 of adding a preservative for conserving molecular components (such as DNA and/or RNA, e.g. viral DNA and/or viral RNA, one or more proteins and/or one or more metabolites). The preservative is preferably added as a liquid in a drop-wise manner. Alternatively or additionally, the non-cellular components (e.g., the plasma or serum) gathered in the extraction member and/or in the flow assay may be dried in a drying step 155, in which a desiccant is positioned adjacent the extraction member and/or of the flow assay.

After step 150 and/or step 155, the separation member and/or the extraction member are transferred from the point-of-care to a laboratory in step 160. It is envisaged that this may be done at ambient conditions (e.g., ambient temperature and/or humidity), but this may depend on the analysis to be performed and/or the analytes to be considered. The transfer step 160 could thus be performed, depending on the circumstances, under ambient conditions. For example, molecules of interest could be detected even if the device is stored for up to 1 week or up to 2 weeks at up to 30 °C, thereby avoiding the need for cooled transport.

As further illustrated in Fig. 1, the method 100 comprises an analyzing step 170 of analyzing the separated cellular components and/or the non-cellular components (e.g., the plasma or serum, or the non-cellular components of the saliva). For this, the cellular components and/or the non-cellular components may be dissolved in a solution and/or washed out from the separation member and the extraction member, respectively. The solution may be an aqueous solution. As evident from the above, the analyzing step 170 preferably is performed after the transfer step 160.

The analyzing step 170 preferably comprises a multi-level analysis in which both the cellular components and the non-cellular components (e.g., the plasma or serum, or the non-cellular components of the saliva) are analyzed by one or more analytical techniques.

In particular, the analysis step 170 preferably comprises at least one first analysis step 180 in which the cellular components are analyzed, for example by one or more genomic techniques (e.g. PCR and/or next-generation sequencing). Such PCR technique allows to identify DNA and/or RNA present in the separated cellular components (e.g., DNA and/or RNA of a virus). For example, blood cells (e.g., lymphocytes) retained in the separation member may be analyzed by genomic techniques, such as PCR. Such PCR analysis may include RT-qPCR and/or dd-PCR. Alternatively or additionally, the first analysis step 180 may comprise analyzing the cellular components retained in the separation member by a proteomics-based method, such as by mass spectrometry (e.g., liquid chromatography mass spectrometry).

The analysis step preferably comprises at least one second analysis step 190 in which the non-cellular components (e.g., the plasma or serum) extracted by the extraction member is analyzed. Such analysis preferably comprises at least one proteomics-based method, such as mass spectrometry (e.g., liquid chromatography mass spectrometry). Such analysis may provide a multi-omic profile. Alternatively or additionally, one or more genomic techniques (e.g. PCR, in particular RT-qPCR and/or dd-PCR, and/or next-generation sequencing) may be performed on the extracted non-cellular components (e.g., the plasma or serum). For example, viral RNA and/or DNA may be detected in the plasma or serum, such as RNA of Sars-CoV-2.

The cellular components and/or the non-cellular components (e.g., the plasma or serum) may also be analyzed by one or more immunofluorescence methods (e.g Luminex technology), for example in order to detect a cytokine storm.

Alternatively or additionally, the device or parts thereof (e.g., the extraction member and/or the separation member) could be used as a biobank for conserving the cellular components and/or the non-cellular components (e.g., the plasma or serum) for later analysis.

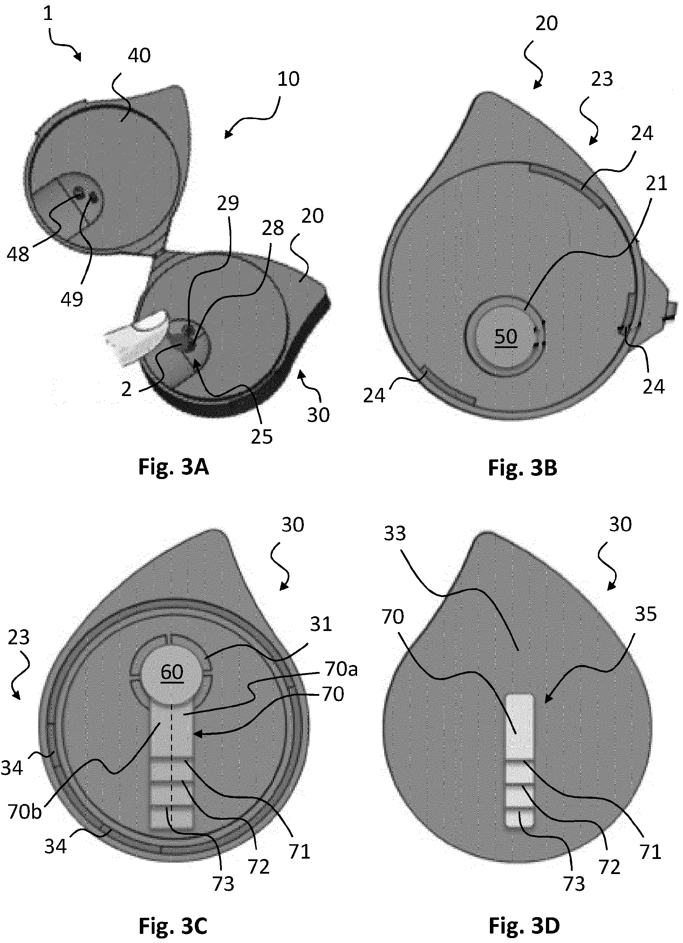

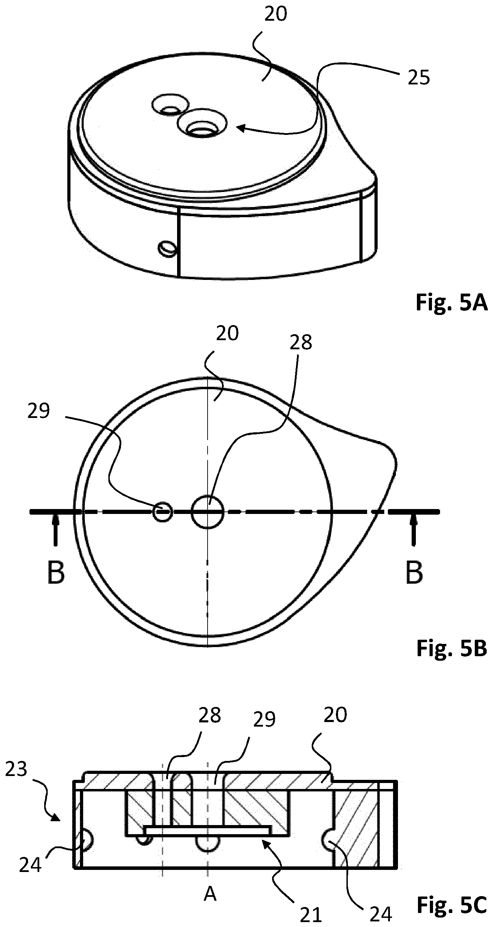

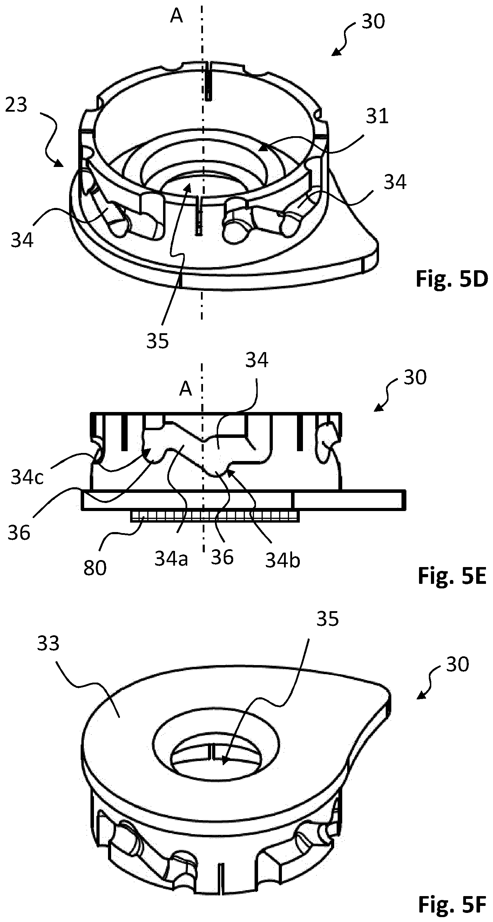

Fig. 2A schematically illustrates a separation member 50 and an extraction member 60 of a device 1 according to the invention (the other components of which are described in more detail hereinafter with reference to Figs. 3A to 3D and Fig. 5) in a first configuration A. In this first configuration A, the separation member 50 is fluidly connected with the extraction member 60 such that liquid (in particular, liquid comprising the non-cellular components, such as the serum or plasma) can flow from the separation member 50 to the extraction member 60. As shown in Fig. 2A, this is, preferably, achieved by contacting a first, lower surface 52 of the separation member 50 with a first, upper surface 61 of the extraction member 60. In this manner a sample 2 (e.g., blood or saliva) applied to a second, upper surface 51 of the separation member 50 may be filtered in the separation member 50 by gravity and/or capillary forces exerted by the extraction member 60. The non-cellular components (e.g., the plasma or serum) provided at the lower surface 52 of the separation member 50 are then extracted by and at least partially gathered in the extraction member 60.

As further illustrated by dashed lines in Fig. 2A, the extraction member 60 may be fluidly coupled to a flow assay 70 (in Fig. 2A, a lateral flow assay is illustrated in an exemplary manner. The flow assay receives at least a portion of the non-cellular components (e.g., the plasma or serum) gathered in the extraction member 60. A flow assay test is then performed on said portion of the non-cellular components for identifying at least one pathogen or other disease, in particular for identifying one or more of nanobodies, and/or antibodies and/or aptamers and/or glycans. For example, the flow assay may identify a serological response against the pathogen (e.g., against a virus), in particular IgA and/or IgM and/or IgG antibodies.

The flow assay 70 may be formed as a unitary structure with the extraction member 60. In particular, the extraction member 60 may form a sample receiving section of the flow assay 70. The flow assay 70 may include a conjugate pad (not shown; e.g., from a cellulose based material) that is provided below the extraction member 60.

Figs. 2B and 2C schematically illustrate a second configuration B of the device wherein the separation member 50 and the extraction member 60 are fluidly uncoupled from each other such that no liquid can flow from the separation member 50 to the extraction member 60. In Fig. 2B, this is achieved by moving the separation member 50 away from the extraction member 60 in a lateral direction (e.g., by a lateral movement and/or a rotation of the members 50, 60 with respect to each other). In Fig. 2C, this is achieved by moving the separation member 50 away from the extraction member 60 in the vertical direction. In the second configuration B, the lower surface 52 of the separation member 50 and the upper surface 61 of the extraction member 60 do not touch each other anymore.

As discussed above, providing the device in such second configuration B is advantageous for applying a preservative 4 for preserving the cellular components gathered in the separation member 50. Moreover, a detergent 6 and/or a buffer 8 may be applied to the extraction member 60 and/or to the flow assay 70.

Fig. 2D shows an alternative manner of severing the fluid connection between the separation member 50 and the extraction member 60. As schematically shown in Fig. 2D, a liquid impervious barrier element 90 may be arranged between the separation member 50 and the extraction member 60 (in particular, between the lower surface 52 of the separation member 50 and the upper surface 61 of the extraction member 60) in order to interrupt the liquid connection and avoid liquid from passing from one member to the other. As the skilled person will appreciate, such barrier element 90 could be employed instead of or in combination with the relative movement of the members 50, 60 described above.

Figs. 3A to 3D show different views of a device 1 according to a first exemplary embodiment of the present invention. The device comprises a housing 10 with a first housing part 20 and a second housing part 30.

As shown in Fig. 3A, the first housing part 20 comprises a sample introduction portion 25 comprising a first through hole 28 though which the sample 2 may be introduced into the device 1. This may be capillary blood dripping from, e.g., a finger, saliva, sputum, urine, or another biofluid. The sample 2 drips onto the first, upper surface 51 of the separation member 50 when applied through the hole 28, in particular when the device 1 is provided in the first configuration A (see Fig. 2A). The sample introduction portion may further include an air hole 29, which also terminates at the separation member 50 and improves evacuation of air when the sample 2 is introduced through the hole 28.

The through hole 28 may also be arranged such that drops of preservative 4 can be applied therethrough when the device 1 is in the second configuration B (see Figs. 2B to 2D).

As further shown in Fig. 3A, the device 1 may also comprise a lid 40. The lid 40 may be arranged to be closed and cover, in particular, the sample introduction portion 25. The lid 40 may comprise a first protrusion 48 configured to protrude into the through hole 28 when the lid 40 is closed. Moreover, the lid 40 may comprise a second protrusion 49 configured to protrude into the air hole 29 when the lid 40 is closed. The lid 40 may be connected by a hinge to the first housing part 20.

Fig. 3B schematically illustrates a bottom view of the first housing part 20. As can been seen in Fig. 3B, the first housing part 20 comprises a separation member holder 21 that may be formed as a rim and/or a cavity in which the separation member 50 is received. In this manner, the separation member 50 is held in and moved with the first housing part 20.

The separation member 50 may be adhesively adhered to the holder 21, in particular along an outer periphery of said separation member 50. This may prevent excess sample volume (e.g., blood or saliva) from the sample 2 from flowing around the separation member 50 and contaminating the non-cellular components (e.g., the plasma or serum) in the extraction member 60.

Fig. 3C schematically illustrates a top view of the second housing part 30. As can been seen in Fig. 3C, the second housing part 30 comprises an extraction member holder 31 that may be formed as a rim and/or a cavity in which the extraction member 60 is received. In this manner, the extraction member 60 is held in and moved with the second housing part 30. As further shown in Fig. 3C, also the flow assay 70 may be received in the second housing part 30. Preferably, the extraction member holder 31 is configured such that the extraction member can be removed therefrom without destroying it (e.g., for washing out the non-cellular components, such as the plasma or serum, in the laboratory).

Figs. 3B and 3C further illustrate a mechanism 23 for shifting the device 1 between the first configuration A (as schematically illustrated in Fig. 2A), in which the separation member 50 is contacted with the extraction member 60, and the second configuration B (see, e.g., Fig. 2B), in which the separation member 50 and the extraction member 60 are moved away from each other, such that a contact is avoided. In the embodiment of the device 1 shown in Figs. 3A to 3D, this is achieved by rotating the first housing part 20 (in which the separation member 50 is being held) with respect to the second housing part 60 (in which the extraction member 60 is being held). In this manner, the separation member 50 is moved from a first position in which it is arranged above and contacted with the extraction member 60 to a second position in which the separation member 50 is not located above and not contacted with the extraction member 60 anymore.

Such mechanism 23 allowing for the rotation of the first and second housing parts 20, 30 with respect to each other is provided in the embodiment of Figs. 3A to 3D by a plurality of protrusions 24 located on an inner peripheral surface of the first housing part 20 that engage with a plurality of grooves 34 provided along an outer peripheral surface of the second housing part 30. Each of the grooves 34 comprises at least one segment extending peripherally around the second housing part 30. Each protrusion 34 is thus able to move in the respective groove 34 along this segment, thereby allowing the user to rotate the first housing part 20 with respect to the second housing part 30.

Since the separation member 50 and/or the extraction member 60 are eccentric with respect to an axis of rotation around which the first and second housing parts 20, 30 are rotated with respect to each other, the mechanism 23 is configured to rotate the separation member 50 and the extraction member 60 away from each other in a horizontal plane.

As the person skilled in the art will appreciate, an alternative bayonet connection could be used, e.g. with one or more grooves provided on the first housing part 20 and one or more respective projections provided on the second housing part 30. Moreover, the housing parts 20, 30 could also be connected and moved with respect to each other also via alternative mechanisms, such as a threaded connection (not shown).

Preferably, the mechanism 23 is configured such that a movement of the first and second housing parts 20, 30 from the second configuration B to the first configuration A is restricted, e.g. by a locking mechanism which can only be overcome by rotating the parts 20, 30 with increased force or cannot by overcome without destruction by rotating the parts 20, 30 backwards. This may prevent a user from moving the device back into the first configuration by mistake.

As further shown in Fig. 3C, the flow assay 70 may provide one or more test lines (which appear when a respective analyte is detected) and at least one control line 73 (which indicates that the flow analysis has been performed), as known in the art. For example, a first test line 71 and a second test line 72 may be provided in addition to the control line 73. In order to analyze various analytes with a single device, the device may also include a first flow assay 70a and a second flow assay 70b, wherein each of these flow assays 70a, 70b is fluidly coupled to the extraction member 60 at least when the device is in the first configuration A and/or in the second configuration B.

Fig. 3D schematically illustrates a bottom view of the second housing part 30. A window 35 is provided in the bottom or base 33 of said second housing part 30. This window 35 allows the user and/or the patient of the device 1 to see the result of the flow assay 70 without having to disassemble the device (which could lead to a contamination of the separation member 50 and/or of the extraction member 60). The window may be provided, for example, by making the respective portion of the base 33 of the second housing part 30 transparent. Preferably, the window 35 is provided by one or more openings in the base 33, which may be helpful for drying the non-cellular components (e.g., the plasma or serum), as discussed hereinbelow.

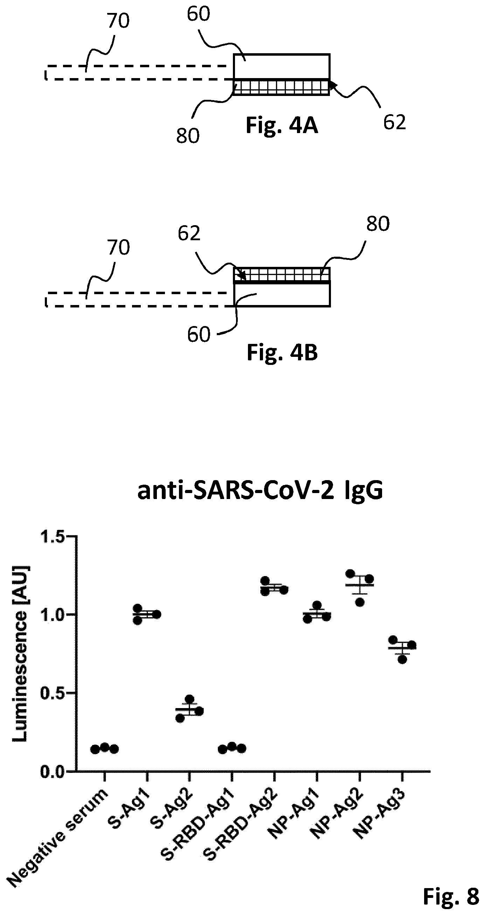

As schematically shown in Figs. 4A and 4B, the device 1 may include at least one desiccant 80. The desiccant 80 preferably is brought into close proximity to the extraction member 60 and/or the flow assay 70, in particular after the non-cellular components have been extracted (step 130 in Fig. 1) and/or after the flow assay has been performed (step 140 in Fig. 1). In this manner, the non-cellular components (e.g., the plasma or serum) may be dried in an efficient manner, without having to dry the entire air in the device 1 and/or having to make the housing 10 air-tight.

One possibility, which is schematically illustrated in Fig. 4A, is to arrange the desiccant 80 below the extraction member 60 and/or below the flow assay 70. This may be achieved, for example, by covering at least part of the window 35 in the base 33 (if provided as one or more openings) with the desiccant 80. The desiccant 80 may be attached to a bottom surface of the base 33 for this purpose, e.g. via an adhesive. This manner of providing the desiccant is particularly simple when the device includes a window 35 in the base 33, e.g. for reading out the result of the flow assay 70, and/or when a fibrous material (e.g. a cellulose-based material) is used as the extraction member 60 and/or the for the flow assay 70. Such arrangement is also particularly advantageous when the separation member 50 and the extraction member 60 are moved away from each other in the vertical direction (see Fig. 2C).

Another possibility, which is schematically illustrated in Fig. 4B, is to arrange the desiccant 80 above the extraction member. This may be achieved by arranging the desiccant 80 in the first housing part 20 at a position that is located above the extraction member 60 when the device 1 is shifted into the second configuration B (or if desired, when a third configuration that is distinct from the first configuration is reached). In particular, the desiccant 80 may be held in the first housing part 20 for this purpose, e.g. in a cavity and/or by a rim provided in the first housing part 20. Such configuration is believed to be particularly advantageous when the device 1 is shifted from the first configuration A to the second configuration B by rotating the first and second housing part 20, 30 with respect to each other (see the embodiment described with reference to Figs. 3A to 3D).

Figs. 5A to 5F schematically illustrate a device according to a second embodiment of the present disclosure. In this embodiment, the device is configured to be shifted between a first configuration and a second configuration by moving the separation member and the extraction member away from each other along an axis A.

In Figs. 5A to 5C, a first housing part 20 of the device is shown. As illustrated therein, the first housing part 20 comprises a sample introduction portion 25 with at least one first through hole 28 for introducing the sample. Optionally, a second through hole 29 may be provided, e.g. for evacuating air.

As best shown in the cross section according to Fig. 5C, the first housing part 20 comprises a holder 21 for receiving the separation member (not shown). The holder 21 may form a cavity in which the separation member is received. The first through hole 28 may be open at the separation member and/or to the cavity of the holder 21. When provided, also the second through hole 29 may be open at the separation member and/or to the cavity of the holder 21.

Figs. 5D to 5F show a second housing part 30. The second housing part 30 comprises a holder 31 for an extraction member and/or a flow assay (not shown). The flow assay in this case may be, in particular, a vertical flow assay arranged below the extraction member. However, also a lateral flow assay could be employed if desired.

Figs. 5C and 5D to 5F further show a mechanism 23 by which the first housing part 20 and the second housing part 30 are connected to each other when the device is assembled. The mechanism is configured for moving the first housing part 20 away from the second housing part 30 along the axis A, which in the exemplary embodiment is the vertical direction.

As most clearly visible in Figs. 5C and 5E, such movement may be provided having one or more protrusion 24 on, e.g., the first housing part 20, that engage with one or more grooves 34 on, e.g., the second housing part 30 (or vice versa). The one or more protrusions 24 may be formed, e.g., along an inner peripheral surface of the second housing part 30, whereas the grooves 34 may be formed along an outer peripheral surface of the second housing part 30 (or vice versa).

In order to provide for a movement of the first housing part 20 with respect to the second housing part 30 along the axis A, the one or more grooves 34 may comprise at least one segment 34a extending in a direction having a directional component along the axis A (e.g., a vertical directional component) as well as a directional component perpendicular to and/or circumferentially around the axis A (e.g., a horizontal directional component). In other words, the portion 34a may extend obliquely to the axis A and/or obliquely to the vertical direction.

When the first housing part 20 is rotated with respect to the second housing part 30, the one or more protrusions 24 move along the portion 34a of the groove (e.g. from a first position 34b to a second position 34c), thereby moving the first housing part 20 away from the second housing part 30 along the axis A. In the example shown, the first housing part 20 will be moved upwards. Thereby, the separation member in the holder 21 is moved away from the extraction member in the holder 31.

The first position 34b may define the first configuration of the device whereas the second position 34c may define the second configuration of the device. The device may be configured to provide a tactile and/or audible feedback indicating to the user that the first configuration and/or the second configuration has been obtained. For example, the one or more grooves 34 may be configured to lock in place the respective protrusion 24 at the first position 34b and/or at the second position 34c, thereby providing a tactile and/or audible feedback. As shown in Fig. 5E, this may be achieved by providing a recess 36 at the first position 34b and/or at the second position 34c.

More generally speaking, the first housing part 20 may be rotatable with respect to the second housing part 30, wherein the rotation causes the first housing part 20 to move relative to the second housing part 30 along the axis A (or vice versa), thereby moving the separation member away from the extraction member.

As further shown in Figs. 5D to 5F, the second housing part 30 may comprise a window window 35 provided in a bottom or base 33 thereof. As indicated above, this window 35 may allow the user and/or the patient of the device to see the result of the flow assay 70 without having to disassemble the device.

Such window may be provided, for example, by making the respective portion of the base 33 of the second housing part 30 transparent. Preferably, the window 35 is provided by one or more openings in the base 33. Such one or more openings are preferably provided below the extraction member and/or below the holder 31.

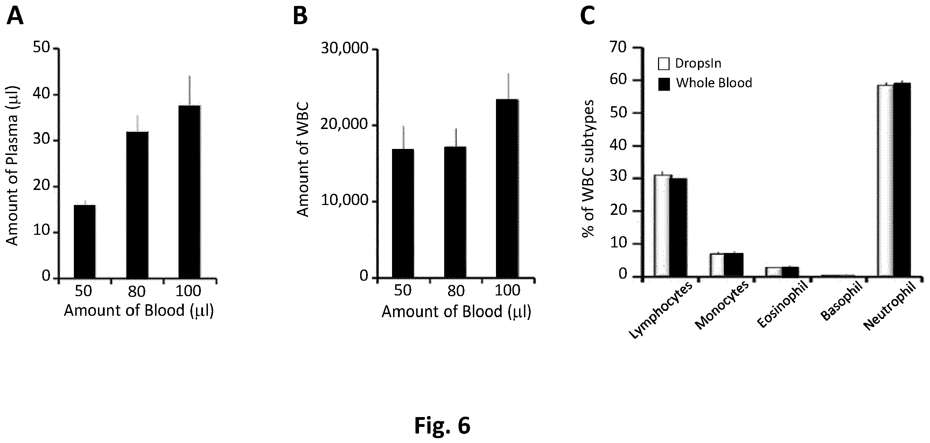

Fig. 6 illustrates the performance of the device disclosed herein (as shown in Figs. 5). In Fig. 6A, the amount of plasma gathered in the extraction member within 3-5 minutes in relation to the amount of blood introduced into the device is shown. Fig. 6B shows the amount of white blood cells (WBC) that were isolated and preserved in the separation member during the gathering of plasma. In Fig. 6C, the isolated and preserved WBC have been characterized by subtypes. As shown, the distribution of subtypes corresponded closely with the distribution in whole blood.

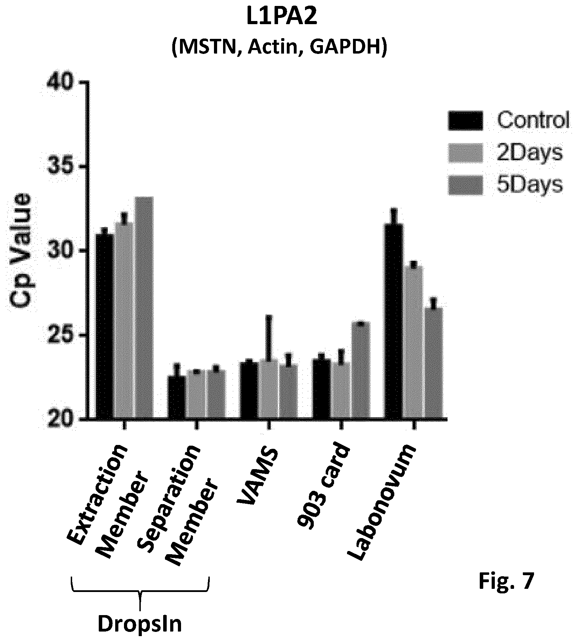

Fig. 7 shows levels of L1PA2 DNA obtained for the device according to the present disclosure (Figs. 5) when compared to different capillary blood sampling solutions. The analysis was performed immediately after separation, 2 days after separation, and 5 days after separation.

L1PA2 is used as a marker for genomic DNA (gDNA) contamination in cell-free DNA profiling from serum samples as its expression is significantly bigger in blood cells (cf. Zhao et al., Performance comparison of blood collection tubes as liquid biopsy storage system for minimizing cfDNA contamination from genomic DNA, Journal of Clinical Laboratory Analysis, 33(2), https://doi.org/10.1002/jcla.22670). In this regard, Fig. 7 shows the Cp (RT-PCR) values obtained after extraction of DNA from the devices referred to on the X axis. The extraction member of the device disclosed herein was shown to have similarly low values of L1PA2 as the ones obtained after centrifugation of liquid capillary blood using Labonovum sampling tubes (obtained from Labnovum, Limmen, Netherlands). However, when the whole blood was stored in the Labonovum tubes 2 or 5 days at room temperature (22 °C) before centrifugation, gDNA contamination in the serum was observed. When using Volumetric Absorptive Microsampling (VAMS) and VAMS and a Whatman^{™} 903^{™} protein saver card, gDNA contamination occurred from the beginning. This is believed to be attributed to the drying and leakage of L1PA2 into serum. This hypothesis seems to be confirmed by a similar contamination observed in the separation member of the inventive device, which gathers the cellular components of blood.

Fig. 8 shows a graph illustrating the detection of anti-SARS-CoV-2 IgG antibodies in the serum of a COVID-19 seroconverted patient by the device according to the present disclosure (see Figs. 5). The serum was preserved in the extraction member of the sampling device for 1 day at +22 °C. The luminescence was measured in an ELISA plate covered by 7 different antigens of the SARS-CoV-2 virus. As shown in the graph, the serum from a non-infected patient shows a residual signal, while the serum of the COVID-19 infected patient shows a very strong reaction to 5 different antigens.

As also indicated above, using such one or more openings, may be advantageous (regardless of whether a flow assay is provided or not) for drying the non-cellular components (e.g., the plasma or serum) gathered in the extraction member. In particular, a desiccant could be applied to the device by covering the one or more openings and/or by inserting the desiccant into said one or more openings. This is schematically illustrated in Fig. 5E where the second housing part 20 is shown standing on and/or adhered to a desiccant 80 such that the opening in the base 33 is covered. A desiccant sheet is shown in this context. However, the desiccant could also be provided in another manner (e.g., as a pellet inserted into the opening or as a granulate on which the device stands, e.g. when inserted into a corresponding transport box). While the invention has been illustrated and described in detail in the drawings and foregoing description, such illustration and description are to be considered illustrative or exemplary and non-restrictive; the invention is thus not limited to the disclosed embodiments. Variations to the disclosed embodiments can be understood and effected by those skilled in the art and practicing the claimed invention, from a study of the drawings, the disclosure, and the appended claims. In particular, features described in the context of particular embodiments could be omitted. Moreover, features disclosed for different embodiments could be combined in a single embodiment. In the claims, the word "comprising" does not exclude other elements or steps, and the indefinite article "a" or "an" does not exclude a plurality and may mean "at least one".

## Claims

1. A device (1) for separation and/or preservation of non-cellular components from a biofluid sample (2) at a point-of-care, wherein said device (1) comprises:
at least one separation member (50) configured to separate the non-cellular components from the sample (2) and to retain cellular components;
at least one extraction member (60) configured to extract the non-cellular components from the separation member (50);
at least one flow assay (70) configured to analyze the non-cellular components extracted by the extraction member (50); and
a housing (10) in which the separation member (50), the extraction member (60) and the flow assay (70) are arranged, the housing (10) including a first housing part (20) and a second housing part (30), wherein the separation member (50) is configured to move with the first housing part (10) and the extraction member (60) is configured to move with the second housing part (20);
wherein the housing (10) is configured to be shifted from a first configuration (A) to a second configuration (B) by rotating the first housing part (10) and the second housing part (20) with respect to each other;
wherein, in the first configuration (A), the separation member (50) and the extraction member (60) are fluidly coupled with each other so that the device (1) is configured for extracting the non-cellular components from the separation member (50) by the extraction member (60); and
wherein, in the second configuration (B), the separation member (50) and the extraction member (60) are fluidly uncoupled from each other for preventing a preservative (4) added to the separation member (50) from reaching the extraction member (60) and/or the flow assay (70).

2. The device (1) of claim 1,
wherein the separation member (50) comprises a separation member upper surface (51) onto which the sample (2) is applied and a separation member lower surface (52) that is opposite the separation member upper surface (51);
wherein the separation member lower surface (52) is in contact with the extraction member (60) when the device (1) is provided in the first configuration (A);
wherein the separation member lower surface (52) is moved away from the extraction member (60) when the device (1) is provided in the second configuration (B).

3. The device (1) of claim 1 or 2,
wherein the device (1) comprises a mechanism (23) for shifting the device (1) between the first configuration (A) and the second configuration (B);
wherein the mechanism (23) is configured to rotate and/or translate the separation member (50) and the extraction member (60) away from each other when the device (1) is moved from the first configuration (1) into the second configuration (2).

4. The device (1) of claim 1, 2 or 3,
wherein the device (1) comprises a mechanism (23) for shifting the device (1) between the first configuration (A) and the second configuration (B);
wherein the mechanism (23) is configured such that a movement of the first housing part (20) and the second housing part (30) from the second configuration (B) to the first configuration (A) is restricted by a locking mechanism which:
can only be overcome by rotating the first and second housing parts (20, 30) backwards with increased force; or
cannot by overcome by rotating the first and second housing parts (20, 30) backwards without destruction of the mechanism (23) and/or at least one of the first and second housing parts (20, 30).

5. The device (1) of claim 3 or 4,
wherein the housing (10) comprises a first housing part (20) and a second housing part (30),
wherein the first housing part (20) and the second housing part (30) are movably connected to each other via the mechanism (23) for shifting the device (1) between the first configuration (A) and the second configuration (B);
wherein the separation member (50) is held in the first housing part (20); and
wherein the extraction member (60) and the flow assay (70) are held in the second housing part (30).

6. The device (1) according to any of the preceding claims,
wherein a first one of the first housing part (20) and the second housing part (30) comprises at least one protrusion (24), wherein the at least one protrusion (24) engages with at least one groove (34) provided on a second one of the first housing part (20) and the second housing part (30), wherein the protrusion (24) moves along the groove (34) when the device (1) is shifted from the first configuration (A) to the second configuration (B); or
wherein the first one of the first housing part (20) and the second housing part (30) comprises a plurality of protrusions (24) that engage with a plurality of grooves (34) provided on the second one of the first housing part (20) and the second housing part (30), wherein the protrusions (24) move along a respective one of the grooves (34) when the device (1) is shifted from the first configuration (A) to the second configuration (B).

7. The device (1) according to any of the preceding claims,
wherein the first housing part (20) comprises a through hole (28);
wherein the through hole (28) is disposed above the separation member (50) when the device (1) is in the first configuration (A) so that the sample (2) can be applied there through and/or wherein the through hole (28) is disposed above the separation member (50) when the device (1) is in the second configuration (B) so that the preservative (4) can be applied there through.

8. The device (1) according to any of the preceding claims, wherein a window (35) through which the flow assay (70) is visible is formed in a bottom of the second housing part (30).

9. The device (1) according to any of the preceding claims, wherein the separation member (50) is configured to separate the non-cellular components from the sample (2) by gravity and/or a capillary force from the extraction member (60).

10. The device (1) according to any of the preceding claims, wherein the flow assay (70) comprises:
at least one first flow assay (70a) for identifying peptides related to a disease by one or more of nanobodies, and/or antibodies and/or aptamers and/or glycans; and/or
at least one second flow assay (70b) for identifying a serological response against the disease, in particular for identifying IgA and/or IgM and/or IgG antibodies against the disease;
preferably wherein the disease is a virus, more preferably wherein the virus is a coronavirus or an influenza virus; more preferably wherein the virus is Sars-CoV-1, Sars-CoV-2 or MERS.

11. A kit comprising the device (1) according to any of claims 1 to 10 and a preservative (4) for preservation of biomolecules, preferably RNA, DNA, proteins, and/or metabolites, gathered in the separation member (50).

12. A method (100) for identifying a disease, preferably an infection by a coronavirus or an influenza virus, more preferably an infection by Sars-CoV-1, Sars-CoV-2 or MERS, the method (100) comprising:
(a) a step (110) of providing a device (1) according to any of claims 1 to 10 at a point-of-care;
(b) a sample application step (120) of applying the sample (2) to the separation member (50) at the point-of-care;
(c) a separating step (130) of separating, by the separation member (50), the non-cellular components from the cellular components, wherein the non-cellular components are extracted from the separation member (50) by the extraction member (60);
(d) a flow assay step (140) of applying at least a portion of the non-cellular components separated by the separation member (50) to a flow assay (70) for identifying peptides of the disease and/or for identifying a serological response against the disease;
(e) an analyzing step (170) of separately analyzing i) the separated cellular components and ii) the non-cellular components by different techniques.

13. The method (100) according to claim 12,
wherein the method (100) further comprises a step (150) of adding a liquid preservative (4) for conserving the molecular integrity of DNA, RNA, proteins and/or metabolites to the separation member (50), wherein the preservative (4) is added after step (c), preferably at the point of care; and/or
wherein the analyzing step (170) comprises a step (180) of analyzing the cellular components retained in the separation member (50) by a genomic technique, preferably by polymerase chain reaction, in order to identify DNA or RNA of both the host and an infectious organism, preferably wherein the cellular components analyzed by polymerase chain reaction comprise lymphocytes retained in the separation member (50).

14. The method (100) according to claim 12 or 13,
wherein the analyzing step (170) comprises a step of detecting viral peptides in the cellular components by mass spectrometry, immune detection and/or ELISA; and/or
wherein the analyzing step (170) comprises a step (190) of detecting viral peptides in the non-cellular components by mass spectrometry.

15. The method according to any of claims 12 to 14, wherein the sample (2) is capillary blood and wherein the capillary blood is applied in a drop-wise manner.

## Patentansprüche

1. Vorrichtung (1) zur Abtrennung und/oder Konservierung von nicht-zellulären Komponenten aus einer Biofluidprobe (2) an einem Versorgungsort, wobei die Vorrichtung (1) aufweist:
zumindest ein Trennelement (50), das dazu ausgelegt ist, die nicht-zellulären Komponenten von der Probe (2) zu trennen und die zellulären Komponenten zurückzuhalten;
zumindest ein Extraktionselement (60), das dazu ausgelegt ist, die nicht-zellulären Komponenten aus dem Trennelement (50) zu extrahieren;
zumindest einen Flow Assay (70), der dazu ausgelegt ist, die durch das Extraktionselement (50) extrahierten nicht-zellulären Komponenten zu analysieren; und
ein Gehäuse (10), in dem das Trennelement (50), das Extraktionselement (60) und der Flow Assay (70) angeordnet sind, wobei das Gehäuse (10) ein erstes Gehäuseteil (20) und ein zweites Gehäuseteil (30) aufweist, wobei das Trennelement (50) dazu ausgelegt ist, sich mit dem ersten Gehäuseteil (10) mitzubewegen und das Extraktionselement (60) dazu ausgelegt ist, sich mit dem zweiten Gehäuseteil (20) m itzubewegen;
wobei das Gehäuse (10) dazu ausgelegt ist, durch Drehen des ersten Gehäuseteils (10) und des zweiten Gehäuseteils (20) relativ zueinander von einer ersten Konfiguration (A) in eine zweite Konfiguration (B) verlagert zu werden;
wobei in der ersten Konfiguration (A) das Trennelement (50) und das Extraktionselement (60) strömungstechnisch miteinander gekoppelt sind, so dass die Vorrichtung (1) zum Extrahieren der nicht-zellulären Komponenten aus dem Trennelement (50) durch das Extraktionselement (60) ausgelegt ist; und
wobei in der zweiten Konfiguration (B) das Trennelement (50) und das Extraktionselement (60) strömungstechnisch voneinander entkoppelt sind, um zu verhindern, dass ein dem Trennelement (50) zugegebenes Konservierungsmittel (4) das Extraktionselement (60) und/oder den Flow Assay (70) erreicht.

2. Vorrichtung (1) nach Anspruch 1,
wobei das Trennelement (50) eine obere Trennelementfläche (51), auf die die Probe (2) aufgetragen wird, und eine untere Trennelementfläche (52) aufweist, die der oberen Trennelementfläche (51) gegenüberliegt;
wobei die untere Trennelementfläche (52) mit dem Extraktionselement (60) in Kontakt steht, wenn die Vorrichtung (1) in der ersten Konfiguration (A) vorliegt;
wobei die untere Trennelementfläche (52) von dem Extraktionselement (60) wegbewegt ist, wenn die Vorrichtung (1) in der zweiten Konfiguration (B) vorliegt.

3. Vorrichtung (1) nach Anspruch 1 oder 2,
wobei die Vorrichtung (1) einen Mechanismus (23) zum Verlagern der Vorrichtung (1) zwischen der ersten Konfiguration (A) und der zweiten Konfiguration (B) aufweist;
wobei der Mechanismus (23) dazu ausgelegt ist, das Trennelement (50) und das Extraktionselement (60) voneinander weg zu drehen und/oder zu verschieben, wenn die Vorrichtung (1) von der ersten Konfiguration (1) in die zweite Konfiguration (2) bewegt wird.

4. Vorrichtung (1) nach Anspruch 1, 2 oder 3,
wobei die Vorrichtung (1) einen Mechanismus (23) zum Verlagern der Vorrichtung (1) zwischen der ersten Konfiguration (A) und der zweiten Konfiguration (B) aufweist;
wobei der Mechanismus (23) derart ausgebildet ist, dass eine Bewegung des ersten Gehäuseteils (20) und des zweiten Gehäuseteils (30) von der zweiten Konfiguration (B) in die erste Konfiguration (A) durch einen Verriegelungsmechanismus eingeschränkt ist, der:
nur durch Zurückdrehen des ersten und des zweiten Gehäuseteils (20, 30) mit erhöhter Kraft überwunden werden kann; oder
nicht durch Zurückdrehen des ersten und des zweiten Gehäuseteils (20, 30) überwunden werden kann, ohne den Mechanismus (23) und/oder zumindest eines aus erstem und zweitem Gehäuseteil (20, 30) zu zerstören.

5. Vorrichtung (1) nach Anspruch 3 oder 4,
wobei das Gehäuse (10) ein erstes Gehäuseteil (20) und ein zweites Gehäuseteil (30) aufweist,
wobei das erste Gehäuseteil (20) und das zweite Gehäuseteil (30) über den Mechanismus (23) zum Verlagern der Vorrichtung (1) zwischen der ersten Konfiguration (A) und der zweiten Konfiguration (B) beweglich miteinander verbunden sind;
wobei das Trennelement (50) in dem ersten Gehäuseteil (20) gehalten ist; und
wobei das Extraktionselement (60) und der Flow Assay (70) in dem zweiten Gehäuseteil (30) gehalten sind.

6. Vorrichtung (1) nach einem der vorhergehenden Ansprüche,
wobei eines aus erstem Gehäuseteil (20) und zweitem Gehäuseteil (30) zumindest einen Vorsprung (24) aufweist, wobei der zumindest eine Vorsprung (24) in zumindest eine Nut (34) eingreift, die an dem anderen aus erstem Gehäuseteil (20) und zweitem Gehäuseteil (30) vorgesehen ist, wobei sich der Vorsprung (24) entlang der Nut (34) bewegt, wenn die Vorrichtung (1) von der ersten Konfiguration (A) in die zweite Konfiguration (B) verlagert wird; oder
wobei eines aus erstem Gehäuseteil (20) und zweitem Gehäuseteil (30) eine Vielzahl von Vorsprüngen (24) aufweist, die in eine Vielzahl von Nuten (34) eingreifen, die an dem anderen aus erstem Gehäuseteil (20) und zweitem Gehäuseteil (30) vorgesehen sind, wobei sich die Vorsprünge (24) entlang der jeweiligen Nuten (34) bewegen, wenn die Vorrichtung (1) von der ersten Konfiguration (A) in die zweite Konfiguration (B) verlagert wird.

7. Vorrichtung (1) nach einem der vorhergehenden Ansprüche,
wobei das erste Gehäuseteil (20) ein Durchgangsloch (28) aufweist;
wobei dann, wenn sich die Vorrichtung (1) in der ersten Konfiguration (A) befindet, das Durchgangsloch (28) oberhalb des Trennelements (50) angeordnet ist, so dass die Probe (2) durch dieses hindurch aufgetragen werden kann, und/oder wobei dann, wenn sich die Vorrichtung (1) in der zweiten Konfiguration (B) befindet, das Durchgangsloch (28) oberhalb des Trennelements (50) angeordnet ist, so dass das Konservierungsmittel (4) durch dieses hindurch aufgetragen werden kann.

8. Vorrichtung (1) nach einem der vorhergehenden Ansprüche,
wobei in einem Boden des zweiten Gehäuseteils (30) ein Fenster (35) ausgebildet ist, durch das der Flow Assay (70) eingesehen werden kann.

9. Vorrichtung (1) nach einem der vorhergehenden Ansprüche,
wobei das Trennelement (50) dazu ausgelegt ist, die nicht-zellulären Komponenten durch Schwerkraft und/oder eine Kapillarkraft von dem Extraktionselement (60) von der Probe (2) zu trennen.

10. Vorrichtung (1) nach einem der vorhergehenden Ansprüche,
wobei der Flow Assay (70) aufweist:
zumindest einen ersten Flow Assay (70a) zur Identifizierung von Peptiden, die mit einer Erkrankung in Verbindung stehen, durch einen oder mehrere Nanokörper und/oder Antikörper und/oder Aptamere und/oder Glykane; und/oder
zumindest einen zweiten Flow Assay (70b) zur Identifizierung einer serologischen Reaktion gegen die Erkrankung, insbesondere zur Identifizierung von IgA- und/oder IgM- und/oder IgG-Antikörpern gegen die Erkrankung;
vorzugsweise wobei die Erkrankung ein Virus ist, weiter bevorzugt wobei das Virus ein Coronavirus oder ein Influenzavirus ist; weiter bevorzugt wobei das Virus Sars-CoV-1, Sars-CoV-2 oder MERS ist.

11. Kit, aufweisend die Vorrichtung (1) nach einem der Ansprüche 1 bis 10 und ein Konservierungsmittel (4) zur Konservierung von Biomolekülen, vorzugsweise RNA, DNA, Proteinen und/oder Metaboliten, die in dem Trennelement (50) gesammelt wurden.

12. Verfahren (100) zur Identifizierung einer Erkrankung, vorzugsweise einer Infektion durch ein Coronavirus oder ein Influenzavirus, weiter bevorzugt einer Infektion durch Sars-CoV-1, Sars-CoV-2 oder MERS, wobei das Verfahren (100) umfasst:
(a) einen Schritt (110) der Bereitstellung einer Vorrichtung (1) nach einem der Ansprüche 1 bis 10 an einem Versorgungsort;
(b) einen Probenauftragungsschritt (120) zum Auftragen der Probe (2) auf das Trennelement (50) am Versorgungsort;
(c) einen Trennschritt (130) zum Trennen der nicht-zellulären Komponenten von den zellulären Komponenten durch das Trennelement (50), wobei die nicht-zellulären Komponenten durch das Extraktionselement (60) aus dem Trennelement (50) extrahiert werden;
(d) einen Durchflusstest-Schritt (140) zum Auftragen zumindest eines Teils der durch das Trennelement (50) abgetrennten nicht-zellulären Komponenten auf einen Flow Assay (70) zur Identifizierung von Peptiden der Erkrankung und/oder zur Identifizierung einer serologischen Reaktion gegen die Erkrankung;
(e) einen Analyseschritt (170) zum getrennten Analysieren von i) den abgetrennten zellulären Komponenten und ii) den nicht-zellulären Komponenten durch verschiedene Techniken.

13. Verfahren (100) nach Anspruch 12,
wobei das Verfahren (100) ferner einen Schritt (150) einer Zugabe eines flüssigen Konservierungsmittels (4) zur Erhaltung der molekularen Unversehrtheit von DNA, RNA, Proteinen und/oder Metaboliten zu dem Trennelement (50) umfasst, wobei das Konservierungsmittel (4) nach Schritt (c), vorzugsweise am Versorgungsort, zugegeben wird; und/oder
wobei der Analyseschritt (170) einen Schritt (180) eines Analysierens der in dem Trennelement (50) zurückgehaltenen zellulären Komponenten durch eine genomische Technik, vorzugsweise durch Polymerasekettenreaktion, umfasst, um DNA oder RNA sowohl des Wirts als auch eines infektiösen Organismus zu identifizieren, vorzugsweise wobei die durch Polymerasekettenreaktion analysierten zellulären Komponenten in dem Trennelement (50) zurückgehaltene Lymphozyten enthalten.

14. Verfahren (100) nach Anspruch 12 oder 13,
wobei der Analyseschritt (170) einen Schritt eines Nachweises viraler Peptide in den zellulären Komponenten durch Massenspektrometrie, Immundetektion und/oder ELISA umfasst; und/oder
wobei der Analyseschritt (170) einen Schritt (190) eines Nachweises viraler Peptide in den nicht-zellulären Komponenten durch Massenspektrometrie umfasst.

15. Verfahren nach einem der Ansprüche 12 bis 14,
wobei die Probe (2) Kapillarblut ist und wobei das Kapillarblut tropfenweise aufgetragen wird.

## Revendications

1. Dispositif (1) pour la séparation et/ou la préservation de composants non cellulaires à partir d'un échantillon de fluide biologique (2) à un point de service, lequel dispositif (1) comprend :
au moins un élément de séparation (50) configuré pour séparer les composants non cellulaires de l'échantillon (2) et retenir les composants cellulaires ;
au moins un élément d'extraction (60) configuré pour extraire les composants non cellulaires de l'élément de séparation (50) ;
au moins un dosage par écoulement (70) configuré pour analyser les composants non cellulaires extraits par l'élément d'extraction (50) ; et
un boîtier (10) dans lequel l'élément de séparation (50), l'élément d'extraction (60) et le dosage par écoulement (70) sont disposés, le boîtier (10) comprenant une première partie de boîtier (20) et une deuxième partie de boîtier (30), dans lequel l'élément de séparation (50) est configuré pour se déplacer avec la première partie de boîtier (10) et l'élément d'extraction (60) est configuré pour se déplacer avec la deuxième partie de boîtier (20) ;
dans lequel le boîtier (10) est configuré pour être basculé d'une première configuration (A) à une deuxième configuration (B) par rotation de la première partie de boîtier (10) et de la deuxième partie de boîtier (20) l'une par rapport à l'autre ;
dans lequel, dans la première configuration (A), l'élément de séparation (50) et l'élément d'extraction (60) sont couplés l'un à l'autre en relation fluidique de façon que le dispositif (1) soit configuré pour extraire les composants non cellulaires de l'élément de séparation (50) par l'élément d'extraction (60) ; et
dans lequel, dans la deuxième configuration (B) l'élément de séparation (50) et l'élément d'extraction (60) sont découplés l'un de l'autre en relation fluidique pour qu'un conservateur (4) ajouté à l'élément de séparation (50) soit empêché d'atteindre l'élément d'extraction (60) et/ou le dosage par écoulement (70).

2. Dispositif (1) selon la revendication 1,
dans lequel l'élément de séparation (50) comprend une surface supérieure d'élément de séparation (51) sur laquelle l'échantillon (2) est appliqué et une surface inférieure d'élément de séparation (52) qui est opposée à la surface supérieure d'élément de séparation (51) ;
dans lequel la surface inférieure d'élément de séparation (52) est en contact avec l'élément d'extraction (60) quand le dispositif (1) est disposé dans la première configuration (A) ;
dans lequel la surface inférieure d'élément de séparation (52) est éloignée de l'élément d'extraction (60) quand le dispositif (1) est disposé dans la deuxième configuration (B).

3. Dispositif (1) selon la revendication 1 ou 2,
lequel dispositif (1) comprend un mécanisme (23) pour faire basculer le dispositif (1) entre la première configuration (A) et la deuxième configuration (B) ;
dans lequel le mécanisme (23) est configuré pour faire tourner et/ou translater l'élément de séparation (50) et l'élément d'extraction (60) à distance l'un de l'autre quand le dispositif (1) est déplacé de la première configuration (1) à la deuxième configuration (2).

4. Dispositif (1) selon la revendication 1, 2 ou 3,
lequel dispositif (1) comprend un mécanisme (23) pour faire basculer le dispositif (1) entre la première configuration (A) et la deuxième configuration (B) ;
dans lequel le mécanisme (23) est configuré de façon qu'un mouvement de la première partie de boîtier (20) et de la deuxième partie de boîtier (30) de la deuxième configuration (B) à la première configuration (A) soit limité par un mécanisme de verrouillage qui :
ne peut être surmonté que par rotation des première et deuxième parties de boîtier (20, 30) vers l'arrière avec une force accrue ; ou
ne peut pas être surmonté par rotation des première et deuxième parties de boîtier (20, 30) vers l'arrière sans destruction du mécanisme (23) et/ou d'au moins l'une parmi les première et deuxième parties de boîtier (20, 30).

5. Dispositif (1) selon la revendication 3 ou 4,
dans lequel le boîtier (10) comprend une première partie de boîtier (20) et une deuxième partie de boîtier (30),
dans lequel la première partie de boîtier (20) et la deuxième partie de boîtier (30) sont connectées de manière mobile l'une par rapport à l'autre via le mécanisme (23) pour faire basculer le dispositif (1) entre la première configuration (A) et la deuxième configuration (B) ;
dans lequel l'élément de séparation (50) est retenu dans la première partie de boîtier (20) ; et
dans lequel l'élément d'extraction (60) et le dosage par écoulement (70) sont retenus dans la deuxième partie de boîtier (30).

6. Dispositif (1) selon l'une quelconque des revendications précédentes,
dans lequel une première parmi la première partie de boîtier (20) et la deuxième partie de boîtier (30) comprend au moins une saillie (24), dans lequel l'au moins une saillie (24) s'engage avec au moins une rainure (34) disposée sur une deuxième parmi la première partie de boîtier (20) et la deuxième partie de boîtier (30), dans lequel la saillie (24) se déplace le long de la rainure (34) quand le dispositif (1) est basculé de la première configuration (A) à la deuxième configuration (B) ; ou
dans lequel la première parmi la première partie de boîtier (20) et la deuxième partie de boîtier (30) comprend une pluralité de saillies (24) qui s'engagent avec une pluralité de rainures (34) disposées sur la deuxième parmi la première partie de boîtier (20) et la deuxième partie de boîtier (30), dans lequel les saillies (24) se déplacent le long de l'une respective des rainures (34) quand le dispositif (1) est basculé de la première configuration (A) à la deuxième configuration (B).

7. Dispositif (1) selon l'une quelconque des revendications précédentes,
dans lequel la première partie de boîtier (20) comprend un trou traversant (28) ;
dans lequel le trou traversant (28) est disposé au-dessus de l'élément de séparation (50) quand le dispositif (1) est dans la première configuration (A) de façon que l'échantillon (2) puisse être appliqué à travers celui-ci, et/ou dans lequel le trou traversant (28) est disposé au-dessus de l'élément de séparation (50) quand le dispositif (1) est dans la deuxième configuration (B) de façon que le conservateur (4) puisse être appliqué à travers celui-ci.

8. Dispositif (1) selon l'une quelconque des revendications précédentes, dans lequel une fenêtre (35) à travers laquelle le dosage par écoulement (70) est visible est formée au fond de la deuxième partie de boîtier (30).

9. Dispositif (1) selon l'une quelconque des revendications précédentes, dans lequel l'élément de séparation (50) est configuré pour séparer les composants non cellulaires de l'échantillon (2) par gravité et/ou une force capillaire provenant de l'élément d'extraction (60).

10. Dispositif (1) selon l'une quelconque des revendications précédentes, dans lequel le dosage par écoulement (70) comprend :
au moins un premier dosage par écoulement (70a) pour identifier des peptides associés à une maladie par un ou plusieurs parmi des nanocorps et/ou des anticorps et/ou des aptamères et/ou des glycanes ; et/ou
au moins un deuxième dosage par écoulement (70b) pour identifier une réponse sérologique contre la maladie, en particulier pour identifier des anticorps IgA et/ou IgM et/ou IgG dirigés contre la maladie ;
de préférence dans lequel la maladie est un virus, mieux encore dans lequel le virus est un coronavirus ou un virus grippal ; mieux encore dans lequel le virus est Sars-CoV-1, Sars-CoV-2 ou MERS.

11. Trousse comprenant le dispositif (1) selon l'une quelconque des revendications 1 à 10 et un conservateur (4) pour la conservation de molécules biologiques, de préférence des ARN, ADN, protéines, et/ou métabolites, rassemblées dans l'élément de séparation (50).

12. Procédé (100) pour identifier une maladie, de préférence une infection par un coronavirus ou un virus grippal, mieux encore une infection par Sars-CoV-1, Sars-CoV-2 ou MERS, le procédé (100) comprenant :
(a) une étape (110) dans laquelle un dispositif (1) selon l'une quelconque des revendications 1 à 10 est fourni à un point de service ;
(b) une étape d'application d'échantillon (120) dans laquelle l'échantillon (2) est appliqué à l'élément de séparation (50) au point de service ;
(c) une étape de séparation (130) dans laquelle les composants non cellulaires sont séparés des composants cellulaires par l'élément de séparation (50), dans laquelle les composants non cellulaires sont extraits de l'élément de séparation (50) par l'élément d'extraction (60) ;
(d) une étape de dosage par écoulement (140) dans laquelle au moins une portion des composants non cellulaires séparés par l'élément de séparation (50) est appliquée à un dosage par écoulement (70) pour l'identification de peptides de la maladie et/ou pour l'identification d'une réponse sérologique contre la maladie ;
(e) une étape d'analyse (170) dans laquelle i) les composants cellulaires séparés et ii) les composants non cellulaires sont analysés séparément par des techniques différentes.

13. Procédé (100) selon la revendication 12,
lequel procédé (100) comprend en outre une étape (150) dans laquelle un conservateur liquide (4) est ajouté à l'élément de séparation (50) pour que l'intégrité moléculaire d'ADN, ARN, protéines et/ou métabolites soit conservée, dans lequel le conservateur (4) est ajouté après l'étape (c), de préférence au point de service ; et/ou
dans lequel l'étape d'analyse (170) comprend une étape (180) dans laquelle les composants cellulaires retenus dans l'élément de séparation (50) sont analysés par une technique génomique, de préférence par une amplification en chaîne par polymérase, afin d'identifier un ADN ou ARN à la fois de l'hôte et d'un organisme infectieux, de préférence dans lequel les composants cellulaires analysés par une amplification en chaîne par polymérase comprennent des lymphocytes retenus dans l'élément de séparation (50).

14. Procédé (100) selon la revendication 12 ou 13,
dans lequel l'étape d'analyse (170) comprend une étape dans laquelle des peptides viraux sont détectés dans les composants cellulaires par spectrométrie de masse, détection immune et/ou ELISA ; et/ou
dans lequel l'étape d'analyse (170) comprend une étape (190) dans laquelle des peptides viraux sont détectés dans les composants non cellulaires par spectrométrie de masse.

15. Procédé selon l'une quelconque des revendications 12 à 14, dans lequel l'échantillon (2) est du sang capillaire et dans lequel le sang capillaire est appliqué en goutte à goutte.
